# FASCICULE DE BREVET EUROPEEN

(11) **EP 2 371 399 B1**
(45) Date de publication et mention de la délivrance du brevet: **07.03.2018**
(21) Numéro de dépôt: 10158610.5
(22) Date de dépôt: 31.03.2010
(51) Int. Cl.: A61L 27/00, C08K 7/02

(54) **Pièce composite pour implantation endo-osseuse et procédé de fabrication d'une telle pièce**
Verbundstoffteil zur Implantation in Knochen und Herstellungsverfahren eines solchen Teils
Composite part for endosseous implantation and method for manufacturing such a part

(43) Date de publication de la demande: 05.10.2011
(73) Titulaire: Ethical Medical Implants SAS, 33700 Merignac (FR)
(72) Inventeur: Cougoulic, Jean-Pierre, 44380, PORNICHET (FR)
(74) Mandataire: Ipside

(56) Documents cités:
- WO-A1-93/16661
- WO-A1-99/61081
- DE-A1- 3 243 861

## Description

L'invention concerne une pièce destinée à être implantée dans un tissu osseux tel qu'un implant dentaire, une prothèse ou un comblement osseux à des fins médicales ou vétérinaires, ladite pièce étant constituée d'un matériau qui, combiné à un procédé d'obtention particulier, permet d'accélérer son ostéo-intégration dans le tissu receveur.

On connait de l'art antérieur différents implants constitués d'un polymère biocompatible dont le procédé d'obtention permet la création d'une texture de surface constituée de micro pores favorisant la colonisation cellulaire par le tissu receveur et accélérant ainsi l'ostéo-intégration dudit implant.

Ces implants de l'art antérieur procurent des résultats très satisfaisants, cependant, l'épaisseur de la couche d'ostéo-intérgration obtenue par ce mécanisme, qui correspond à la profondeur des micropores de surface est de l'ordre de 1000 nanomètres (1 µm). Or, il est généralement admis qu'une épaisseur d'interpénétration du tissu et de l'implant plus importante, au moins comprise entre 1µm et 10µm, est préférable et ceci sera d'autant plus vrai que les caractéristiques d'élasticité entre l'implant et le tissu receveur seront différentes. Une telle interpénétration accrue sera donc particulièrement recherchée lorsque l'implant est renforcé, notamment par des fibres et plus particulièrement au début du processus d'osté-integration lorsque la corticale en formation ne présente pas encore un module d'élasticité comparable à celui de l'implant.

Par ailleurs, de telles textures micro-poreuses de surface sont difficiles, voire impossibles à obtenir par des procédés économiques de fabrication de l'implant tel que le moulage par injection.

L'invention a pour objet de résoudre ces insuffisances de l'art antérieur en proposant un implant et un procédé d'obtention économique de celui-ci permettant d'augmenter la profondeur d'interpénétration entre l'implant et le tissu osseux receveur.

A cette fin l'invention propose une pièce adaptée à une implantation endo-osseuse in vivo telle que définie dans la revendication 1. On entend par fibres des microfibres, des nanofibres ou des nanotubes dont le ratio longueur sur épaisseur est supérieur à 10. Les microfibres sont des fibres dont l'épaisseur est de l'ordre du micromètre ou micron, c'est-à-dire dont l'épaisseur est sensiblement comprise entre 10⁻⁶ et 10⁻⁵ mètres. Les nanofibres et les nanotubes sont des fibres dont l'épaisseur est de l'ordre du nanomètre, c'est à dire sensiblement comprise entre 10⁻⁹ et 10⁻⁸ mètres.

Le délaminage des fibres dans la couche surfacique permet de créer des interstices qui agissent comme des conduits et par capillarité dans l'épaisseur de cette couche pour y transporter les liquides organiques accélérant ainsi la colonisation cellulaire de celle-ci.

L'invention peut être mise en oeuvre selon des modes de réalisation avantageux décrits ci-après lesquels peuvent être utilisés seuls ou en combinaison.

Avantageusement le liant thermoplastique est constitué de polyétheréthercétone (PEEK) dont les propriétés de biocompatibilité sont connues. Avantageusement également, les fibres sont constituées d'un polymère de la famille des polyamides aromatiques qui présentent également d'excellentes propriétés de biocompatibilité alliées à des caractéristiques mécaniques élevées. Plus particulièrement des fibres poly(amide-imide) dont la température de transition vitreuse est proche de la température de moulage par injection du PEEK permettent par leur facilité de déformation en cours d'injection une répartition homogène des fibres dans l'implant même lorsque celles-ci sont relativement longues.

L'effet de conduction des fibres délaminées dans la couche surfacique permet d'obtenir une épaisseur de ladite couche d'au moins 2µm, c'est à dire nettement supérieure à ce qu'il est possible d'obtenir avec des implants obtenus par injection plastique comportant des micro pores en surface sans effet de délaminage.

Avantageusement le matériau constituant l'implant peut comprendre, en plus des fibres, une charge de composants à base de calcium et de phosphore. Ces composés résorbables favorisent l'ostéo-intégration et la cicatrisation.

Le matériau constituant la pièce implantable peut également contenir une charge de zéolites. Ceux-ci favorisent les liaisons électrostatiques avec le milieu d'implantation et un accrochage ionique avec celui-ci. Cette charge contribue en outre à la radio-opacité du matériau.

L'invention concerne également un procédé tel que défini dans la revendication 9. Le procédé d'injection plastique permet de produire de manière économique ce type d'implant en cote finie dès l'opération de moulage et en quantité importante. Il permet en outre d'orienter les fibres par le flux de matière pénétrant dans le moule et ainsi d'obtenir un effet de renforcement optimal même pour les implants de forme complexe. Le traitement physico-chimique combinant l'effet chimique des bains et mécanique des ultrasons permet à la fois de décaper / dérocher la surface de l'implant, afin d'en éliminer toute pollution relative au procédé de moulage par injection, et de produire, dans une couche surfacique, les délaminages des fibres aptes à produire l'effet de conduction recherché.

Afin d'obtenir une pièce comprenant, en plus des fibres, des composés à base de calcium et de phosphates, le procédé d'obtention d'un granulat, ou compound, comprend les étapes consistant à :
- fabriquer un premier granulat, ou compound, en mélangeant, par extrusion et granulation, le liant polymère avec des composés introduits sous forme de poudre amorphe ; et
- Mélanger ce premier granulat avec des fibres au cours d'une seconde opération d'extrusion granulation de sorte à former le granulat utilisé pour l'opération de moulage par injection.
Avantageusement la charge de zéolites peut également être introduite lors de la fabrication du premier granulat.

La charge de fibres est comprise en 5% et 15% en masse du mélange. Cette proportion offre à la fois un effet de renforcement important de la pièce finale tout en permettant son obtention par le procédé d'injection et en permettant le mélange des fibres au liant polymère par extrusion granulation ou « compounding », que celui-ci ait ou non été préalablement chargé par des composés contenant du calcium et/ou des zéolites. Le granulat, ou compound, pour la fabrication par injection plastique d'un implant renforcé par des fibres comprend :
- un liant polymère de polyétheréthercétone (PEEK) ;
- une charge de 10% à 20% en masse de composés contenant du calcium et des zéolites ;
- une charge de 5% à 15% de fibres constituées de poly(amide-imide).

Ce type de granulat peut être utilisé directement pour la fabrication par injection plastique, selon l'étape b) du procédé de fabrication d'une pièce selon l'un des modes de réalisation de l'invention.

Après moulage, la pièce est soumise à un décapage dans une succession de bains soumis aux ultrasons, dans le but de la rendre apte à une implantation in vivo et pour y créer avantageusement une couche surfacique favorisant l'ostéo-intégration dans le milieu receveur.

Avantageusement le décapage est réalisé dans une succession de bains comprenant, dans l'ordre :
- un passage dans un bain soumis aux ultrasons apte à réduire les particules contenant du fer ;
- une passage dans un solvant du liant soumis aux ultrasons, inerte vis-à-vis des fibres.

Le premier bain permet d'éliminer la pollution surfacique par les particules métalliques issues de la presse d'injection et du moule. Le deuxième bain, en ne dissolvant que le liant, permet, avec l'action conjuguée des ultrasons, de créer les décohésions ou délaminages entre les fibres et la matrice dans une couche surfacique. L'ordre des bains est important dans la mesure ou l'acide peut également avoir une action réductrice vis-à-vis des fibres et / ou vis-à-vis de la charge de composés contenant du calcium ou des zéolites présents en surface. En réalisant l'attaque acide en premier, l'action suivante du solvant a pour effet de refaire apparaître ces composés en surface. L'opération de décapage, plus particulièrement adapté au mode de réalisation pour lequel le matériau constituant l'implant comprend des fibres et une charge de zéolites et de composés contenant du calcium dans une matrice PEEK, comprend, dans l'ordre, le passage dans les bains suivants :
- Acide chlorhydrique
- Acétone
- Eau oxygénée

Séparés par des rinçages dans un bain d'eau également soumis aux ultrasons.

L'invention sera maintenant plus précisément décrite dans le cadre de modes de réalisation préférés, nullement limitatifs, représentés sur les figures 1 à 3, dans lesquelles :
- la figure 1 représente de manière schématique et de face un exemple de pièce destinée à une implantation endo-osseuse ;
- la figure 2 montre un détail en coupe en vue de dessus de la même pièce ;
- la figure 3 représente un détail en coupe de la surface de la pièce.

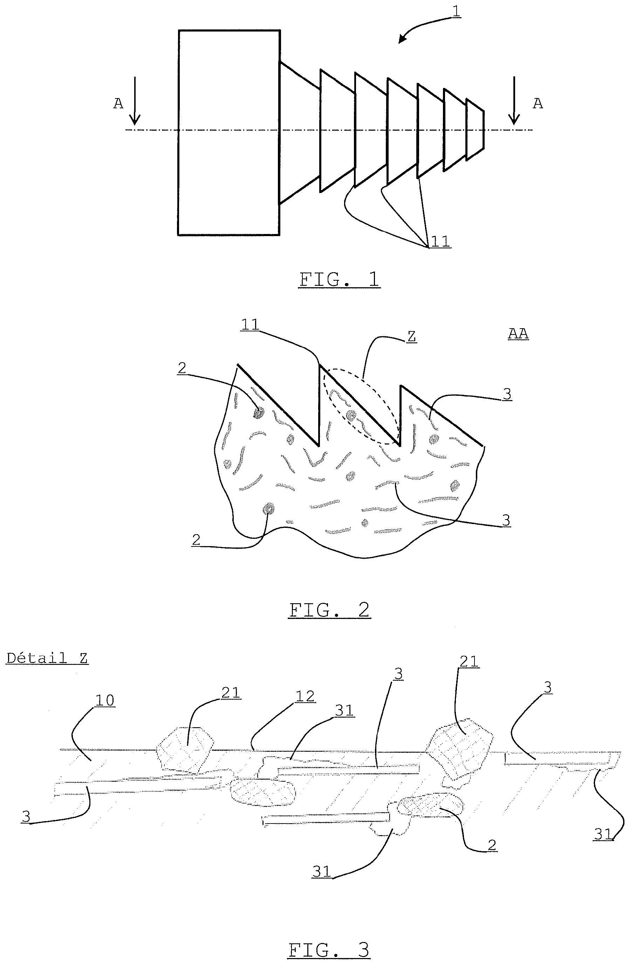

Figure 1, un exemple d'implant (1) présentant une forme complexe peut être obtenu de manière économique par un procédé de moulage par injection plastique. Ledit implant peut optionnellement comprendre des reliefs tels que des stries (11) aptes à favoriser son accrochage mécanique primaire dans un logement tel qu'un alésage pratiqué dans le tissu osseux receveur. Ces stries ou reliefs d'accrochage primaire on une dimension de l'ordre du millimètre. Ledit implant est constitué majoritairement d'un polymère thermoplastique présentant de bonne caractéristiques de biocompatibilité et apte à être mis en oeuvre par des techniques de moulage par injection. A titre d'exemple non limitatif celui-ci peut être constitué de polyétheréthercétone ou PEEK tel que distribué commercialement par la société VICTREX® sous la dénomination VICTREX® PEEK 150G®. Alternativement le liant peut être constitué d'un matériau comprenant à la fois du PEEK, des charges de composés contenant du calcium et des zéolites tel que le matériau décrit dans le brevet français FR2722694 ou le brevet américain US5872159.

Figure 2, selon ce dernier exemple, le matériau constituant l'implant comprendra des particules (2) de composés contenant du calcium tels que du phosphate tricalcique et des particules de zéolites tels que le dioxyde de titane (TiO₂) et des fibres (3) lesquelles sont orientées par le flux de matière lors du procédé d'injection. Les particules de composés, ont, si on les assimile à des sphères, un diamètre de l'ordre du micron (10⁻⁶ mètres). Les fibres sont avantageusement constituées d'un poly(amide-imide), telles que des fibres disponibles commercialement sous la dénomination KERMEL® TECH auprès de la société KERMEL®, 20 rue Ampère, F-68027 Colmar. Selon un exemple de réalisation à base de microfibres, celles-ci ont un diamètre d'environ 7µm pour une longueur d'environ 700µm (0,7 mm).

Figure 3, l'observation de la surface à une échelle encore plus fine révèle la présence du polymère liant en PEEK (10), des composés contenant du calcium sous forme cristallisée en surface (21) et amorphe (2) à distance de la surface libre (12). Les composés contenant du calcium sont résorbables au contact du milieu receveur. Les fibres (3) présentent des décohésions (31) ou délaminages avec le liant (10) lesquels permettent l'introduction dans l'épaisseur d'une couche surfacique des liquides interstitiels et des cellules du milieu receveur, le cas échéant, après résorption des composés (21) en surface.

Selon un exemple de réalisation, l'implant est obtenu par une première étape visant à l'obtention d'un granulat mélangeant :
- 80% en poids de PEEK
- 10 % en poids de phosphate tricalcique (Ca₃PO₄)
- 10 % en poids de dioxyde de titane (TiO₂)

L'ensemble étant mélangé par extrusion à une température comprise entre 340°C et 400°C .

Par granulation de cette extrusion on obtient un premier granulat, lequel est mélangé avec 10 % en masse de fibres Poly(amide-imide) de type KERMEL® TECH selon le même procédé d'extrusion granulation.

Le second granulat ainsi obtenu est utilisé pour le moulage par injection plastique de l'implant. Le moulage se déroule à une température comprise entre 340°C et 400°C sous une pression comprise entre 70 et 140MPa, le moule étant chauffé à une température supérieure à la température de transition vitreuse du PEEK soit une température de préchauffage du moule d'environ 160°C.

La température de transition vitreuse des fibres de type KERMEL® étant de 340°C celles-ci sont déformables à la température d'injection ce qui leur permet de suivre le flux d'écoulement de la matière et de se répartir de manière homogène, dans le granulat lors de l'opération d'extrusion granulation et dans la pièce lors de l'opération de moulage par injection.

A l'issue de l'opération de moulage, l'implant est soumis à une série de bain d'attaque chimique / décapage soumis aux ultrasons. A titre d'exemple le protocole suivant procure de bons résultats pratiques, les ultrasons étant appliqués avec une fréquence de 42 KHz :
- HCl 30 %: 35 minutes
- H₂O: 10 minutes (ou rinçage)
- C₃H₆O (acétone): 35 minutes à température d'ébullition de l'acétone
- Séchage de l'implant par évaporation de l'acétone
- H₂O₂ 30 %: 35 minutes
- NaClO: 35 minutes
- H₂O:10 minutes (ou rinçage)
L'implant est ensuite plongé, toujours sous ultrasons dans des produits stérilisants :
- GIGASEPT® 12 %: 35 minutes
- H₂O ppi: 35 minutes
Le bain dans la solution GIGASEPT® étant facultatif.

Avantageusement, l'implant est ensuite inséré dans une gaine de stérilisation pour son passage en autoclave. Il subit alors un cycle de stérilisation à une température de l'ordre de 135°C pendant 10 minutes, sous une pression de l'ordre de 2150 hPa. Cette opération de stérilisation par autoclave contribue à la fonction de décapage surfacique; elle peut être associée à un traitement par oxyde d'éthylène ou par rayons gamma. En outre, elle favorise la cristallisation des particules de phosphate tricalcique en surface.

L'application de ce traitement surfacique à un implant ne contenant que des composés de phosphate de calcium et du dioxyde de titane dans une matrice en PEEK permet d'obtenir une épaisseur de couche surfacique active d'environ 1µm. Le même traitement appliqué à un implant de forme identique mais constitué d'un matériau comprenant en plus 10% de fibres de poly(amide-imide) permet d'obtenir une épaisseur de couche surfacique active de 3,6µm.

La description ci-avant illustre clairement que par ses différentes caractéristiques et leurs avantages, la présente invention atteint les objectifs qu'elle s'était fixés. En particulier, elle permet d'obtenir un implant moulé par injection et renforcé, comprenant une couche surfacique d'ostéo-intégration d'épaisseur au moins trois fois supérieure à ce qu'il est possible d'obtenir sans renforcement.

## Revendications

1. Pièce (1) adaptée à une implantation endo-osseuse in vivo constituée d'un matériau comprenant :
- un liant organique thermoplastique (10), et
- une charge fibreuse comprise entre 5% et 15% en masse, et comprenant une couche surfacique d'une épaisseur supérieure ou égale à 2000 nanomètres, pour l'introduction dans l'épaisseur de ladite couche des liquides interstitiels et des cellules du milieu receveur, **caractérisée en ce que** les fibres (3) de la charge fibreuse, comprises dans ladite couche surfacique présentent majoritairement des délaminages ou décohésions (31) avec le liant (10) sur toute ou partie de leur longueur, de sorte à créer des interstices aptes à transporter les liquides organiques dans l'épaisseur de ladite couche surfacique par capillarité.

2. Pièce selon la revendication 1, dans laquelle la charge fibreuse est constituée de nanofibres ou de nanotubes.

3. Pièce selon la revendication 1, dans laquelle la charge fibreuse est constituée de microfibres.

4. Pièce selon la revendication 1, dans laquelle le liant (1) est constitué de polyétheréthercétone

5. Pièce selon la revendication 1, dans laquelle les fibres (3) sont constituées d'un polymère de la famille des polyamides aromatiques.

6. Pièce selon la revendication 5, dans laquelle les fibres (3) sont constituées de poly(amide-imide).

7. Pièce selon la revendication 1, constituée d'un matériau comprenant en outre une charge de composants (2) à base de calcium et de phosphate.

8. Pièce selon la revendication 7, dans laquelle le matériau dont elle est constituée comprend en outre une charge de zéolites.

9. Procédé pour la fabrication d'une pièce selon l'une quelconque des revendications 1 a 6 **caractérisé en ce qu'**il comprend les étapes consistant a :
a) mélanger par extrusion et granulation un polymère thermoplastique et une charge de fibres; et
b) mouler la pièce par injection dans un moule comprenant une empreinte de forme appropriée a partir du granulat obtenu a l'étape a);et
c) soumettre l'ébauche obtenue à l'étape b) à des bains de décapage sous ultrasons durant un temps approprié pour produire un délaminage des fibres dans une couche surfacique comprenant, dans l'ordre, le passage dans les bains suivants soumis aux ultrasons:
- Acide chlorhydrique
- Acétone
- Eau oxygénée
séparés par des rinçages dans un bain d'eau également soumis aux ultrasons

## Patentansprüche

1. Teil (1), das für eine enossale Implantation in vivo ausgelegt ist, bestehend aus einem Material, das Folgendes umfasst:
- ein organisches thermoplastisches Bindemittel (10) und
- einen faserförmigen Füllstoff zwischen 5 und 15 Massen-%
und umfassend eine Oberflächenschicht einer Dicke von größer als oder gleich 2000 Nanometer, für das Einbringen in die Dicke der Schicht der interstitiellen Flüssigkeiten und Zellen der Empfängerumgebung,
**dadurch gekennzeichnet, dass** die Fasern (3) des faserförmigen Füllstoffs, die in der Oberflächenschicht enthalten sind, größtenteils Entlaminierungen oder Ablösungen (31) von dem Bindemittel (10) über ihre gesamte oder einen Teil ihrer Länge aufweisen, so dass Zwischenräume gebildet werden, die organische Flüssigkeiten mittels Kapillarwirkung in die Dicke der Oberflächenschicht transportieren können.

2. Teil nach Anspruch 1, wobei der faserförmige Füllstoff aus Nanofasern oder Nanoröhrchen besteht.

3. Teil nach Anspruch 1, wobei der faserförmige Füllstoff aus Mikrofasern besteht.

4. Teil nach Anspruch 1, wobei das Bindemittel (1) aus Polyetheretherketon besteht.

5. Teil nach Anspruch 1, wobei die Fasern (3) aus einem Polymer aus der Familie der aromatischen Polyamide bestehen.

6. Teil nach Anspruch 5, wobei die Fasern (3) aus Poly(amid-imid) bestehen.

7. Teil nach Anspruch 1, das aus einem Material besteht, das außerdem einen Füllstoff aus Komponenten (2) auf der Basis von Calcium und Phosphat umfasst.

8. Teil nach Anspruch 7, wobei das Material, aus dem es besteht, außerdem einen Zeolithenfüllstoff umfasst.

9. Verfahren zur Herstellung eines Teils nach einem der Ansprüche 1 bis 6, **dadurch gekennzeichnet, dass** es die folgenden Schritte umfasst:
a) Mischen eines thermoplastischen Polymers und eines Faserfüllstoffs mittels Extrusion und Granulation und
b) Formen des Teils durch Einspritzen in eine Form, die einen Hohlraum der entsprechenden Form umfasst, ausgehend von dem in Schritt a) erhaltenen Granulat und
c) Unterziehen des in Schritt b) erhaltenen Rohlings Beizbädern unter Ultraschall für einen angemessenen Zeitraum, dass eine Entlaminierung der Fasern in einer Oberflächenschicht hervorgerufen wird, umfassend in dieser Reihenfolge das Hindurchleiten durch die folgenden, Ultraschallwellen unterworfenen Bäder:
- Salzsäure
- Aceton
- Wasserstoffperoxid,
die durch Spülungen in einem ebenfalls Ultraschallwellen unterworfenen Wasserbad voneinander getrennt sind.

## Claims

1. Part (1) suitable for *in vivo* endosseous implantation, composed of a material that comprises:
- a thermoplastic organic binder (10), and
- a fibrous filler of between 5 and 15% by weight,
and comprising a surface layer with a thickness greater than or equal to 2000 nanometres, for introduction of the interstitial liquids and cells of the recipient medium into the thickness of said layer,
**characterized in that** the majority of the fibres (3) of the fibrous filler, which are included in said surface layer, have areas of delamination (31) from the binder (10) over all or part of their length, in such a way as to create interstices for transporting the organic liquids within the thickness of said surface layer by capillary action.

2. Part according to Claim 1, in which the fibrous filler is composed of nanofibres or nanotubes.

3. Part according to Claim 1, in which the fibrous filler is composed of microfibres.

4. Part according to Claim 1, in which the binder (1) is composed of polyether ether ketone.

5. Part according to Claim 1, in which the fibres (3) are composed of a polymer from the family of aromatic polyamides.

6. Part according to Claim 5, in which the fibres (3) are composed of polyamide-imide.

7. Part according to Claim 1, composed of a material that additionally comprises a filler of components (2) based on calcium and phosphate.

8. Part according to Claim 7, in which the material of which it is composed additionally comprises a zeolite filler.

9. Method for manufacturing a part according to any one of Claims 1 to 6, **characterized in that** it comprises the steps consisting of:
a) mixing a thermoplastic polymer and a fibrous filler by extrusion and granulation; and
b) injection-moulding the part in a mould comprising a cavity of suitable shape, using the granulate obtained in step a); and
c) subjecting the blank obtained in step b) to ultrasonic pickling baths for a suitable period of time in order to delaminate the fibres from a surface layer comprising, in order, passing the blank through the following baths subjected to ultrasound:
- hydrochloric acid
- acetone
- aqueous hydrogen peroxide solution
separated by rinsing in a bath of water likewise subjected to ultrasound.
